# EUROPÄISCHE PATENTANMELDUNG

(11) **EP 3 244 353 A1**
(43) Veröffentlichungstag der Anmeldung: **15.11.2017**
(21) Anmeldenummer: 16168897.3
(22) Anmeldetag: 10.05.2016
(51) Int. Cl.: G06Q 10/06

(54) **PRODUKTIONSMODUL ZUR DURCHFÜHRUNG EINER PRODUKTIONS-FUNKTION AN EINEM PRODUKT**

(71) Anmelder: Siemens Aktiengesellschaft, 80333 München (DE)
(72) Erfinder: Zahorcak, Vladimir, 900 32 Borinka (SK)

(57) **Zusammenfassung**

Die Erfindung betrifft ein Verfahren zur Simulation einer industriellen Fertigungsanlage, wobei mehrere Produktionsmodule (1,..,n) zu einem Anlagenlayout (AL) der zu simulierenden Fertigungsanlage zusammen gestellt werden, wobei als Produktionsmodule (1,..,n) tragbare, mobile elektronische Geräte mit einer Anzeigeeinheit (D) verwendet werden, wobei auf den Geräten eine Simulationssoftware (S) zum Ablauf gebracht wird, und das die Simulationssoftware (S) auf dem jeweiligen Gerät durch eine Benutzereingabe auf fertigungsspezifische Merkmale des dieses Gerät repräsentierenden Produktionsmoduls (1, ..,n) angepasst wird, wobei ein erstes Produktionsmodul (1) durch eine Simulation auf einem ersten Gerät und ein zweites Produktionsmodul (2) durch eine Simulation auf einem zweiten Gerät repräsentiert wird, wobei zur Simulation eines Fertigungsablaufs dem ersten Produktionsmodul (1) ein computergeneriertes Produktmodell (40) übergeben wird und entsprechend der fertigungsspezifischen Merkmale des ersten Produktionsmoduls (1) eine erste Produktions-Funktion auf das Produktmodell (40) angewendet wird und das Produktmodell (40) an das zweite Produktionsmodul (2) übergeben wird, wobei in dem zweiten Produktionsmodul (2) auf das Produktmodell (40) eine zweite Produktions-Funktion angewendet wird.

## Beschreibung

Die vorliegende Erfindung betrifft ein Verfahren zur Simulation einer industriellen Fertigungsanlage, wobei mehrere Produktionsmodule zu einem Anlagenlayout der zu simulierenden Fertigungsanlage zusammen gestellt werden.

Derartige Verfahren zur Simulation sind aus dem Stand der Technik bekannt. So offenbart die Master Arbeit "DEPlaTa - A Digitally Enhanced Planning Table for Rough Factory Layouts" von Nico Herbig vom September 2015 beispielsweise Intuplan, welches ein intuitives Planungswerkzeug für ein Anlagenlayout ist.

Es ist ein Nachteil des Standes der Technik, dass relativ viel Mitwirkung eines Benutzers bei der Erstellung eines derartigen Anlagenlayout erforderlich ist, so müssen zum Beispiel Produktionsmodelle mit einem 3-D Drucker gedruckt undplatziert werden. Zusätzlich müssen sie noch mit einem QR-Code versehen und fotografiert werden.

Daher ist es eine Aufgabe der vorliegenden Erfindung, die Simulation einer industriellen Anlage zu vereinfachen.

Diese Aufgabe wird gelöst durch ein Verfahren mit den Merkmalen des vorliegenden Patentanspruchs 1.

Bei dem Verfahren zur Simulation einer industriellen Fertigungsanlage, wobei mehrere Produktionsmodule zu einem Anlagenlayout der zu simulierenden Fertigungsanlage zusammen gestellt werden, werden als Produktionsmodule tragbare, mobile elektronische Geräte mit einer Anzeigeeinheit verwendet, welche es für einen Benutzer sehr einfach machen beispielsweise Tablett-PCs oder Smart-Phones einfach aneinander zu legen um ein zu entwickelndes Anlagenlayout ab zu bilden. Auf den Geräten wird eine Simulationssoftware zum Ablauf gebracht, und die Simulationssoftware auf dem jeweiligen Gerät lässt sich durch eine Benutzereingabe auf fertigungsspezifische Merkmale des dieses Gerät repräsentierenden Produktionsmoduls, beispielsweise ein Roboter, ein Fließband, eine Drehbank, eine Testanlage, usw., anpassen, wobei ein erstes Produktionsmodul durch eine Simulation auf einem ersten Gerät und ein zweites Produktionsmodul durch eine Simulation auf einem zweiten Gerät repräsentiert wird, wobei zur Simulation eines Fertigungsablaufs dem ersten Produktionsmodul ein computergeneriertes Produktmodell übergeben wird und entsprechend der fertigungsspezifischen Merkmale des ersten Produktionsmoduls eine erste Produktions-Funktion auf das Produktmodell angewendet wird und das Produktmodell an das zweite Produktionsmodul übergeben wird, wobei in dem zweiten Produktionsmodul auf das Produktmodell eine zweite Produktions-Funktion angewendet wird.

Um das Anlagenlayout, beispielweise auf einem großen Tisch abzubilden geht man wie folgt vor:
a) Positionieren des ersten Produktionsmoduls an eine erste Position, wobei durch diese erste Position des ersten Produktionsmoduls ein Koordinatenursprung gebildet wird, wobei jedes tragbare, mobile elektronische Gerät eine linke Anlegeseite, eine rechte Anlegeseite, eine obere Anlegeseite und eine untere Anlegeseite aufweist und
b) abhängig von den fertigungsspezifischen Merkmalen des ersten Produktionsmoduls werden auf der Anzeigeeinheit des ersten Produktionsmoduls mögliche Anlegeseiten, also wo ein virtuelles Produkt das Produktionsmodul verlässt und zum nächsten Produktionsmodul wandert, mittels eines Berührobjektes angezeigt, das Berührobjekt ist dann beispielweise ein Pfeil, auf den man zum auslösen einer Funktion mit den Finger tippen kann,
c) Positionieren des zweiten Produktionsmoduls als Nachbar zu dem ersten Produktionsmodul, wobei auf der Anzeigeeinheit des zweiten Produktionsmoduls abhängig von den fertigungsspezifischen Merkmalen des zweiten Produktionsmoduls ebenfalls mögliche Anlegeseiten mittels weiterer Berührobjektes angezeigt werden, anlegen einer durch ein Berührobjekt markierten Anlegeseite des zweiten Geräts an eine durch ein Berührobjekt markierten Anlegeseite des ersten Geräts,
d) Betätigen beider Berührobjekte von den jetzt zueinander gewandten Anlegeseiten, wodurch eine zweite Position des zweiten Produktionsmoduls bezogen auf den Koordinatenursprung festgelegt wird,
f) Positionieren der weiteren Produktionsmodule analog zu dem wie unter Schritt c) und d) beschrieben bis das gewünschte Anlagenlayout erreicht ist.

Nachdem alle Geräte positioniert sind und vorzugsweise über eine drahtlose Kommunikation miteinander verbunden sind, könnte eine zusammenhängende simulierte Produktion eines virtuellen Produktes starten. Das virtuelle Produkt oder das virtuelle Werkstück durchläuft dann den simulierten Produktionsprozess und wandert damit von einem mobilen Gerät zu dem nächsten mobilen Gerät, wobei das virtuelle Werkstück jeweils auf den Displays der mobilen Geräte sichtbar wird. An den Geräten könnte auch angezeigt werden, welche Produktions-Funktion grade auf das virtuelle Werkstück angewendet wird. Das virtuelle Werkstück bzw. das virtuelle Produkt kann somit entlang des Weges von einem ersten Produktionsmodul bis zu einem letzten Produktionsmodul verfolgt werden.

Ein Benutzer kann leicht nachprüfen, ob die simulierte industrielle Fertigungsanlage das geplante Produkt korrekt fertigt. Auch kann er an den Geräten Informationen über Key Performance Indicatoren (KPI) bzw. Leistungskennzahlen ablesen, z.B. Produktionszeit oder Gesamtenergieverbrauch. Die Simulation der industriellen Fertigungsanlage kann für verschiedene Produkte gestartet werden und kann auch flexible durch neupositionieren der Geräte angepasst oder verändert werden, um verschieden Anlagenlayouts zu testen.

Ein ein Produktionsmodul repräsentierendes mobiles Gerät ist zur Durchführung der Produktions-Funktion an dem Produktmodell ausgebildet und eingerichtet und weiterhin ausgebildet zur Kopplung mit einem zweiten Produktionsmodul. Das zweite Produktionsmodul ist wiederum zur Durchführung der zweiten Produktions-Funktion an dem Produktmodell ausgebildet und eingerichtet. Dabei ist in einer ersten Speichereinrichtung des ersten Produktionsmoduls eine erste SelbstbeschreibungsInformation bezüglich Eigenschaften des ersten Produktionsmoduls gespeichert und weiterhin umfasst das zweite Produktionsmodul eine zweite Selbstbeschreibungs-Information bezüglich Eigenschaften des zweiten Produktionsmoduls. Das erste Produktionsmodul ist zum Übertragen der ersten Selbstbeschreibungs-Information an das zweite Produktionsmodul und zum Empfang der zweiten Selbstbeschreibungs-Information vom zweiten Produktionsmodul ausgebildet und eingerichtet. In der Speichereinrichtung des jeweiligen Produktionsmoduls ist weiterhin eine Port-Information bezüglich der Kopplung mit weiteren Produktionsmodulen gespeichert.

Über das Vorliegen der Selbstbeschreibungs-Informationen des ersten und zweiten Produktionsmoduls und der Möglichkeit, diese gegenseitig aneinander zu übertragen und das gleichzeitige Vorliegen einer Port-Information bezüglich der Kopplung mit dem zweiten Produktionsmodul im ersten Produktionsmodul, liegt sowohl eine Information über die Fähigkeiten der Module als auch eine Information über eine Kopplung mit dem zweiten Produktionsmodul im Produktionsmodul vor. Damit kann das erste Produktionsmodul beispielsweise in eine Lage versetzt werden, zu der Behandlung oder Bearbeitung eines Produkts mit relativ wenig oder auch ohne eine Benutzereinwirkung mit dem zweiten Produktionsmodul zusammen zu arbeiten. Auf diese Weise wird der Aufbau einer beispielsweise die zwei Produktionsmodule umfassenden zu simulierenden Fertigungsanlage vereinfacht.

Die Produktionsmodule können verschiedenste mechanische, elektromechanische oder elektronische Einrichtungen nachbilden, die zur Behandlung, Bewegung, Verarbeitung und/oder Bearbeitung eines Gegenstands, Werkstücks, einer Flüssigkeit oder vergleichbaren Produkten, Baugruppen oder Materialien eingerichtet und ausgebildet sind. Produktionsmodule können beispielsweise Werkzeugmodule, Werkmaschinen (z.B. zum Fräsen, Bohren, Stanzen, Pressen, o.ä.) oder ähnliche Werkzeuge, Geräte oder Maschinen oder auch Elemente davon sein. Weiterhin können Produktionsmodule auch zum zumindest unter Anderem Transport von Produkten, Baugruppen oder Materialien ausgebildet und eingerichtet sein, beispielsweise als Förderband, Kran, Roboterarm, Pumpe oder Ähnliches. Weiterhin können Produktionsmodule auch zur Lagerung oder Zufuhr entsprechender Produkte ausgebildet und eingerichtet sein (z.B. umfassend ein Regalsystem, Tank, o.ä.). Das Produktionsmodul kann auch beispielsweise zum Erhitzen oder auch sonstigen Bearbeiten von Werkstücken, Baugruppen und/oder festen, flüssigen oder gasförmigen Materialien ausgebildet und eingerichtet sein, z.B. als Ofen, Kessel, Ventil, Rührer, o.Ä..

Das Produktionsmodul selbst kann wiederum aus mehreren Unter-Modulen aufgebaut sein und/oder beispielsweise eine oder mehrere mechanische und/oder elektronische Untereinheiten nachbilden.

Ein Produktionsmodul kann beispielsweise als eine Steuereinheit oder einen Controller zur Steuerung der Produktionsfunktion und/oder der in der vorliegenden Beschreibung in Bezug auf das Produktionsmodul beschriebenen Verfahren, Vorgänge und Ermittlungen ausgebildet und eingerichtet sein. Weiterhin kann das Produktionsmodul ein oder mehrere Kommunikationsschnittstellen sowie ein oder mehrere Speichereinrichtungen zur Speicherung von Daten und/oder Informationen umfassen. Zur Durchführung der Produktions-Funktion kann das Produktionsmodul zudem entsprechende mechanische, elektrische, elektronische und/oder elektromechanische oder optische Komponenten nachbilden.

Insbesondere kann das Produktionsmodul ein sogenanntes "Cyber-Physical-Systems" (CPS) oder ein Teil davon simulieren. So kann das Produktionsmodul beispielsweise ein sogenanntes "Cyber-Physical-Module" (CPM) oder ein "Cyber-Physical-Production-Module" (CPPM) für eine Simulation nachbilden.

Unter einer Produktions-Funktion wird im Rahmen der vorliegenden Beschreibung allgemein jeder Vorgang verstanden, der im Rahmen der Produktion, Herstellung, Verarbeitung, Behandlung oder Bearbeitung eines Gegenstands, eines Materials oder eines Stoffes durchgeführt wird beziehungsweise durchgeführt werden kann. Eine Produktions-Funktion kann dabei z.B. jeder mögliche Arbeitsschritt bezüglich eines beliebigen Produktes von den Ausgangsstoffen bis zum fertigen Endprodukt sein.

Beispielsweise kann eine Produktions-Funktion jegliche Art von Material-Bearbeitung (z.B. Fräsen, Bohren, Schleifen, Pressen, Lackieren, Gießen, Pumpen, Erhitzen, Bewegen, Öffnen, Schließen, usw.), jegliche Art von Transport oder Bewegung oder Handhabung eines Gegenstands, einer Baugruppe, eines Materials oder eines Stoffes sein oder derartige Vorgänge umfassen. Weiterhin kann die Produktions-Funktion beispielsweise eine Lagerung, Diagnose, Prüfung, optische Aufnahme, Vermessung, Bestimmung einer Form, Lage oder Größe oder vergleichbare Funktionalitäten sein, beziehungsweise derartige Funktionalitäten umfassen.

Ein Produkt kann beispielsweise als ein mechanisches, optisches, elektromechanisches, elektronisches oder vergleichbares Produkt beziehungsweise Produktmodell nachgebildet sein. Weiterhin kann das Produkt beispielsweise als ein Werkstück, eine Baugruppe, ein festes, flüssiges oder gasförmiges Material, eine feste, flüssige oder gasförmige Chemikalie oder Ähnliches als ein Produktmodell nachgebildet sein.

Im Rahmen der vorliegenden Beschreibung wird der Begriff "Produkt" als eine abstrakte Beschreibung eines im Rahmen einer Produktion oder Bearbeitung durchaus veränderlichen Gegenstands verwendet. Ein "Produkt" im Sinne der vorliegenden Beschreibung kann sich im Laufe eines Produktionsprozesses, beispielsweise durch die Einwirkung von Produktions-Funktionen, in seiner äußeren oder inneren Erscheinung oder Ausgestaltung durchaus verändern.

Die Kopplung des ersten Produktionsmoduls mit dem zweiten Produktionsmodul kann derart ausgebildet und eingerichtet sein, dass die erste Produktions-Funktion des ersten Produktionsmoduls und die zweite Produktions-Funktion des zweiten Produktionsmoduls zusammenwirken können beziehungsweise zusammenwirken. Ein derartiges Zusammenwirken von Produktions-Funktionen kann beispielsweise eine gemeinsame Bearbeitung eines Produkts, eine Bearbeitung und einen Transports eines Produkts oder auch eine Übergabe eines Produkts von einer zu einer weiteren Transporteinheit sein. Zwei Produktionsmodule können dazu beispielsweise in einer geeigneten geometrischen Anordnung befindlich sein und z.B. elektronisch derart gekoppelt, dass das Zusammenwirken der Produktions-Funktionen ermöglicht beziehungsweise durchgeführt wird oder durchführbar ist.

Die Kopplung des ersten Produktionsmoduls mit dem zweiten Produktionsmodul oder allgemein die Kopplung zweier benachbarten Produktionsmodule kann beispielsweise als eine kommunikative Kopplung über entsprechende drahtgebundene oder drahtlose Kommunikationsschnittstellen (z.B. über Ehternet, Profinet, Profibus, Feldbusse, WLAN, Bluetooth, NFC usw.) ausgebildet und eingerichtet sein beziehungsweise eine derartige kommunikative Kopplung umfassen.

Das zweite Produktionsmodul kann beispielsweise entsprechend dem in der vorliegenden Beschreibung beschriebenen ersten Produktionsmodul ausgebildet und eingerichtet sein. Weiterhin können die Produktionsmodule jeweils mit weiteren Produktionsmodulen gekoppelt sein, die wiederum jeweils entsprechend einem Produktionsmodul beziehungsweise einem ersten Produktionsmodul gemäß der vorliegenden Beschreibung ausgebildet und eingerichtet sein können. Dabei kann die Kopplung mit den jeweils anderen Produktionsmodulen auch wie in der vorliegenden Beschreibung näher erläutert ausgebildet und eingerichtet sein.

Die Selbstbeschreibungs-Information bezüglich der Eigenschaften des Produktionsmoduls kann beispielsweise die verschiedensten Informationen bezüglich der Produktions-Funktion des Produktionsmoduls umfassen. Insbesondere kann es beispielsweise eine Kennung beziehungsweise Charakterisierung der Funktionalität beziehungsweise Funktionalitäten umfassen, welche in der Produktions-Funktion realisiert ist bzw. sind. Weiterhin können sie Informationen über be- oder verarbeitbare Materialien beziehungsweise Gegenstände, Informationen über Größen-, Form-, Gewichts- oder ähnliche Vorgaben oder Voraussetzungen, Informationen über einen oder mehrere Bearbeitungsbereiche des Produktionsmoduls, Informationen über Qualitätskriterien, -ergebnisse und/oder Voraussetzungen bezüglich der Produktionsfunktion beziehungsweise des entsprechenden Arbeitsergebnisses oder Produkts oder ähnliche Informationen bezüglich der Produktions-Funktion umfassen.

Die Selbstbeschreibungs-Information kann zudem Information über sonstige Eigenschaften des Produktionsmoduls, wie beispielsweise eine Größe, eine Geometrie, eine Lage, eine Identifikations-Kennung, ein Aufbau, eine Konfiguration, verfügbare Services und Funktionaltitäten, angeschlossene Geräte, Module und/oder Baugruppen, verfügbare Steuer- und sonstige Kommandos sowie verfügbare Kommunikations-Schnittstellen, entsprechende Kommunikations-Parameter (MAC-Adresse o.Ä.) und/oder eine Zustands-Information bezüglich des Produktionsmoduls umfassen.

Die Selbstbeschreibungs-Information bezüglich der Eigenschaften des zweiten Produktionsmoduls sowie auch bezüglich weiterer in der vorliegenden Beschreibung genannter Produktionsmodule kann entsprechend den vorstehenden Ausführungen ausgestaltet und eingerichtet sein.

Das Übertragen der Selbstbeschreibungs-Information zwischen den Produktionsmodulen kann beispielsweise als drahtgebundene und/oder drahtlose Kommunikation erfolgen beziehungsweise ausgestaltet und eingerichtet sein. Eine derartige Übertragung kann beispielsweise über Ethernet, Feldbusse, WLAN, Bluetooth, NFC, optisch oder Ähnliches erfolgen beziehungsweis ausgestaltet und eingerichtet sein. Bei der Kopplung des zweiten Produktionsmoduls mit dem ersten kann beispielsweise vorgesehen sein, dass die zweite Selbstbeschreibungs-Information des zweiten Produktionsmoduls an das erste Produktionsmodul gesendet wird. Weiterhin kann auch die erste Selbstbeschreibungs-Information des ersten Produktionsmoduls an das zweite Produktionsmodul gesendet werden. Auch ein Austausch der Stellenbeschreibungs-Informationen zwischen Produktionsmodul und zweitem Produktionsmodul kann vorgesehen sein.

Die im ersten Produktionsmodul gespeicherte Port-Information bezüglich der Kopplung mit dem zweiten Produktionsmodul kann beispielsweise eine Information über die zweite Produktions-Funktion des zweiten Produktionsmoduls umfassen. Weiterhin kann die Port-Information eine Information über eine Wechselwirkung der Produktionsfunktion des Produktionsmoduls und der zweiten Produktionsfunktion des zweiten Produktionsmoduls umfassen. Derartige Informationen können beispielsweise Informationen über einen Übergabe-Bereich oder Wechselwirkungs-Bereich sein, in welchem sich beispielsweise ein Produkt befinden muss oder kann, um eine derartige Wechselwirkung der Produktions-Funktionen zu ermöglichen.

Derartige Wechselwirkungen können beispielsweise eine gemeinsame Bearbeitung eines Produkts, eine Bearbeitung eines in einem Transport-Modul befindlichen Produkts oder die Übergabe zwischen zwei Transport-Modulen sein.

Weiterhin kann die Port-Information auch Informationen über weitere, über das zweite Produktionsmodul erreichbare Module, Produktions-Funktionen und/oder Funktionalitäten umfassen. Derartige Informationen über solche weiteren Module beziehungsweise Produktionsfunktionen können den vorstehend genannten Informationen bezüglich des zweiten Produktionsmoduls entsprechen.

Weiterhin kann das Produktionsmodul unmittelbar noch mit einem oder mehreren weiteren Produktionsmodulen gemäß der vorliegenden Beschreibung gekoppelt sein, wobei weiterhin vorgesehen sein kann, dass für jedes der unmittelbar mit dem Produktionsmodul verbundenen weiteren Produktionsmodule eine entsprechende Port-Information bezüglich der Kopplung mit dem jeweiligen Produktionsmodul im Produktionsmodul gespeichert wird oder ist. Diese Port-Information kann entsprechend der vorstehend beschriebenen Port-Information bezüglich der Kopplung mit dem zweiten Produktionsmodul ausgestaltet und eingerichtet sein.

Die Ermittlung der Port-Information bezüglich der Kopplung mit dem zweiten oder auch weiteren Produktionsmodulen kann beispielsweise im Produktionsmodul erfolgen, oder auch in einem externen Rechner oder einer vergleichbaren Einrichtung, und dann an das Produktionsmodul übertragen werden. Unter der "Ermittlung" wird hier beispielsweise eine reine Datenextraktion aus entsprechenden Nachrichten oder das Auslesen entsprechender Informationsdaten, aber auch die Verarbeitung übermittelter oder übertragener Daten oder Informationen verstanden.

In einer vorteilhaften Ausgestaltung kann die Port-Information bezüglich der Kopplung mit dem zweiten Produktionsmodul eine Information über einen räumlichen Wechselwirkungsbereich des Produktionsmoduls mit dem zweiten Produktionsmodul umfassen, wobei der räumliche Wechselwirkungsbereich dadurch gekennzeichnet ist, dass sowohl die Produktions-Funktion des ersten Produktionsmoduls, als auch die zweite Produktions-Funktion des zweiten Produktionsmoduls an dem Produkt wirken kann, wenn es sich im Wechselwirkungsbereich befindet.

Die Information über den Wechselwirkungsbereich kann es den Produktionsmodulen z.B. ermöglichen, mit reduzierter oder auch ohne Benutzermitwirkung zusammen zu arbeiten, da dem Produktionsmodul bekannt ist, in welchem räumlichen Bereich sich das zu bearbeitende oder zu handhabende Produkt oder Material befinden muss, um sowohl selbst, als auch durch das zweite Produktionsmodul bearbeitet oder gehandhabt zu werden. Auf diese Weise wird der Aufbau und Betrieb eines entsprechenden Produktionssystems weiter vereinfacht.

Insbesondere kann vorgesehen sein, dass der Wechselwirkungsbereich auf Grund der Selbstbeschreibungsinformation des ersten Produktionsmoduls sowie der zweiten Selbstbeschreibungsinformation des zweiten Produktionsmoduls ermittel wird oder ermittelbar ist. Diese Ermittlung kann beispielsweise im Produktionsmodul, im zweiten Produktionsmodul und/oder in einer externen Rechner oder ähnlichen Einrichtung erfolgen.

Zu diesem Zweck kann beispielsweise in den jeweiligen Selbstbeschreibungs-Informationen jeweils ein Arbeitsbereich der jeweiligen Produktionsmodule abgelegt sein und dann der Wechselwirkungsbereich über die Kenntnis der jeweiligen Arbeitsbereiche sowie der relativen Lage der Produktionsmodule zueinander ermittelt werden. Dabei kann die Information zur relativen Lage der Produktionsmodule beispielsweise vorgegeben oder vorgebbar sein oder auch automatisch oder durch einen Benutzer ermittelt werden.

Die Port-Information kann dann beispielsweise eine Information über eine Geometrie des Wechselwirkungsbereichs und/oder eine Position des Wechselwirkungsbereichs umfassen. Dabei können Geometrie und/oder Position in entsprechenden Modul-Koordinaten oder einem anderen Koordinatensystem, beispielsweise einem für beide Produktionsmodule gemeinsamen Koordinatensystem gespeichert sein.

Für eine spätere reale Produktion mit einer realen Fertigungsanlage und realen Produktionsmodulen ist es sehr vorteilhaft, dass wenn die Simulation einen Zielzustand erreicht hat, die während der Simulation gewonnenen Parameter und die Einstellungen zumindest einer der folgenden Informationen zu übernehmen:
- die Selbstbeschreibungsinformation,
- die Service-Information,
- die Konfigurations-Information,
- die Fähigkeits-Information,
- die Befehls-Information,
- die Zustands-Information,
zu speichern und für die spätere reale Produktion zu verwenden.

Betrachtet man den kompletten Speicherbereich der oben aufgeführten Informationen und Parameter als einen Software-Stack, so ist es sehr sinnvoll diesen Software-Stack bzw. die einzelnen Software-Stacks der unterschiedlichen Produktionsmodule in spätere reale Cyper-Physical-Systeme zu übernehmen.

Wegen der Wiederverwendbarkeit des Software-Stacks ist es ebenso möglich reale Cyper-physikalische-Module, welche hinsichtlich des Software-Stacks genauso aufgebaut sind, in ein zu simulierendes Anlagenlayout zu integrieren oder umgekehrt und auf diese Weise eine Mischung aus realen und simulierten Modulen für eine Entwicklung/Weiterentwicklung und Inbetriebnahme von realen Fertigungsanlagen zu nutzen.

Insbesondere kann eine Position des Wechselwirkungsbereichs beispielsweise in entsprechenden Modul-Koordinaten oder auch einem weiteren Koordinatensystem, beispielsweise einem gemeinsamen Koordinatensystem der Module, angegeben und/oder abgelegt sein. Gleiches gilt für Geometrie, beispielsweise die räumliche Ausgestaltung, des Wechselwirkungsbereichs.

In einer vorteilhaften Ausgestaltung kann die im Produktionsmodul gespeicherte Port-Information bezüglich der Kopplung mit dem zweiten Produktionsmodul Informationen bezüglich Eigenschaften von mit dem zweiten Produktionsmodul unmittelbar und/oder mittelbar verbundenen weiteren Produktionsmodulen umfassen. Insbesondere kann die im Produktionsmodul gespeicherte Port-Information bezüglich der Kopplung mit dem zweiten Produktionsmodul Informationen bezüglich Produktions-Funktionen von mit dem zweiten Produktionsmodul unmittelbar und/oder mittelbar verbundenen Produktionsmodulen umfassen.

Auf diese Weise lässt sich der Aufbau und der Betrieb einer Fertigungsanlage aus derartigen Produktionsmodulen weiter vereinfachen, indem bei in einem bestimmten Produktionsmodul befindlichen Produkt bereits über die Port-Information bezüglich eines gekoppelten zweiten Produktionsmoduls erkennbar ist, welche weiteren Produktionsmodule und/oder Produktions-Funktionen über eine Kopplung beziehungsweise Zusammenwirkung mit dem zweiten Produktionsmodul verfügbar oder erreichbar sind.

Dabei können Eigenschaften von mit dem zweiten Produktionsmodul verbundenen weiteren Produktionsmodulen solche Eigenschaften sein, wie sie beispielsweise bezüglich Selbstbeschreibungs-Informationen eines Produktionsmoduls in der vorliegenden Beschreibung an anderer Stelle näher ausgeführt sind.

Insbesondere kann die Port-Information bezüglich der Kopplung mit dem zweiten Produktionsmodul Eigenschaften bezüglich aller mit dem zweiten Produktionsmodul unmittelbar und/oder mittelbar verbundenen weiteren Produktionsmodule umfassen. Weiterhin können die gespeicherten Informationen bezüglich der Eigenschaften der weiteren Produktionsmodule auch auf bestimmte Kategorien von Produktionsmodulen, technischen Einschränkungen, räumlichen Einschränkungen oder auch funktionalen Einschränkungen umfassen.

Dabei sind mit dem zweiten Produktionsmodul unmittelbar verbundene weitere Produktionsmodule solche Module, welche über eine unmittelbare Kopplung beziehungsweise Anbindung an das zweite Produktionsmodul verfügen. Mittelbar verbundene weitere Produktionsmodule sind solche, welche keine unmittelbare Kopplung zum zweiten Produktionsmodul aufweisen, sondern wiederum über zu andere Produktionsmodule vom zweiten Produktionsmodul aus erreichbar sind.

Zwei Produktionsmodule sind unmittelbar verbunden, wenn die Module beispielsweise "wirksam" verbunden sind, wenn also beispielsweise ihre Produktionsfunktionen gemäß der vorliegenden Beschreibung - wie bereits erläutert - zusammenwirken beziehungsweise zusammenwirken können. Insbesondere sind sie unmittelbar verbunden, wenn sie gemäß der vorliegenden Beschreibung gekoppelt sind oder werden. So können beispielsweise zwei verbundene Transportmodule einen durchgehenden Transport eines Produkts mit einer entsprechenden Übergabe zwischen den Modulen ermöglichen. Eine mit einem weiteren Modul verbundene Bearbeitungseinheit kann dann beispielsweise eine Bearbeitung eines im anderen Modul befindlichen Produkts oder eine mit dem anderen Modul gemeinsame Bearbeitung ermöglichen.

Weiterhin kann die im Produktionsmodul gemäß der vorliegenden Beschreibung gespeicherte Selbstbeschreibungs-Information eine Konfigurations-Information bezüglich einer Lage und/oder Ausgestaltung des Produktionsmoduls umfassen.

Die Selbstbeschreibungs-Information kann auch eine Fähigkeits-Information bezüglich verfügbarer Funktionen und Services des Produktionsmoduls umfassen, wobei diese beispielsweise eine Information über die Produktions-Funktion umfassen kann.

Weiterhin kann die Selbstbeschreibungs-Information auch eine Befehls-Information bezüglich vom Produktionsmodul ausführbarer oder verstehbarer Befehle sowie einstellbarer oder eingestellter Parameter umfassen.

Zudem kann die Selbstbeschreibungs-Information auch eine Zustandsinformation bezüglich eines Arbeitszustands umfassen.

Dabei kann ein Arbeitszustand beispielsweise einen aktuellen Betriebszustand (voll funktional aktiv, teilweise funktional aktiv, inaktiv, Notbetrieb, o.Ä.) oder auch Informationen bezüglich aufgetretener Fehler und Warnungen oder Ähnlichem umfassen. Die Zustandsinformation kann weiterhin eine Information über ein im bzw. am Produktionsmodul vorhandenes Produkt (z.B. eine entsprechende Produkt-ID, ein aktueller Bearbeitungs-Zustand, eine aktuelle Position innerhalb der Produktionsmoduls, o.ä.) umfassen.

Die Konfigurations-Information des Produktionsmoduls kann beispielsweise eine Position, eine funktionale Ausgestaltung und/oder eine geometrische Ausgestaltung des Produktionsmoduls umfassen. Weiterhin kann die Konfigurations-Information des Produktionsmoduls auch einen verfügbaren und/oder zugänglichen räumlichen Arbeitsbereich oder ein physikalisches und sonstiges Umfeld (z.B. benachbarte Module, Maschinen, Sicherheitsbereiche, o.Ä.) umfassen.

Die im Produktionsmodul gespeicherte Port-Information bezüglich der Kopplung mit dem zweiten Produktionsmodul kann Informationen bezüglich Eigenschaften eines mit dem zweiten Produktionsmodul gekoppelten dritten Produktionsmodul umfassen, wobei das dritte Produktionsmodul mit dem zweiten Produktionsmodul gekoppelt ist, im dritten Produktionsmodul eine dritte Selbstbeschreibungsinformation bezüglich Eigenschaften des dritten Produktionsmoduls gespeichert oder speicherbar ist und das dritte Produktionsmodul zur Durchführung einer dritten Produktions-Funktion an dem Produkt ausgebildet und eingerichtet ist.

Dabei kann die Information bezüglich der Eigenschaften des mit dem zweiten Produktionsmodul gekoppelten dritten Produktionsmoduls eine Information bezüglich der dritten Produktions-Funktion des dritten Produktionsmoduls umfassen und weiterhin die Kopplung des dritten Produktionsmoduls mit dem zweiten Produktionsmodul gemäß der in der vorliegenden Beschreibung erläuterten Kopplung zwischen Produktionsmodul und zweiten Produktionsmodul ausgebildet und eingerichtet sein. Die Selbstbeschreibungsinformation des dritten Produktionsmoduls beziehungsweise die dritte Produktions-Funktion dieses Moduls kann weiterhin gemäß den entsprechenden Informationen beziehungsweise Funktionen des Produktionsmoduls oder zweiten Produktionsmoduls gemäß der vorliegenden Beschreibung ausgebildet und eingerichtet sein.

Informationen über das dritte Produktionsmodul können beispielsweise an das Produktionsmodul gelangen, indem z.B. die Selbstbeschreibungsinformation beziehungsweise Teile davon über das zweite Produktionsmodul an das Produktionsmodul übertragen werden und dort im Rahmen der gespeicherten Port-Information insgesamt, in Auszügen oder nach entsprechender Be- oder Verarbeitung gespeichert werden.

Weiterhin kann auch vorgesehen sein, dass auch weitere oder auch alle mittelbar oder unmittelbar mit dem zweiten Produktionsmodul verbundene Produktionsmodule derartige Selbstbeschreibungs-Informationen umfassen und diese über das zweite Produktionsmodul zum Produktionsmodul übertragbar sind beziehungsweise übertragen werden. Auf diese Weise kann eine entsprechende Information zu diesen weiteren Produktionsmodulen ebenfalls in der Port-Information bezüglich der Kopplung mit dem zweiten Produktionsmodul gespeichert werden. Auf diese Weise können Informationen über Funktionalitäten, Eigenschaften, Zustände oder weitere Parameter von mehreren oder auch allen über das zweite Produktionsmodul weiterhin zugänglichen Produktionsmodule ebenfalls im Produktionsmodul verfügbar sein.

Dies ermöglicht eine weiterhin vereinfachte Simulation beziehungsweise einen weiterhin vereinfachten Entwurf eines Anlagenlayouts aus derartigen Produktionsmodulen, da auf diese Weise eine verbesserte Planung über weitere Produktionsschritte, die mit einem im Produktionsmodul befindlichen Produkt durchgeführt werden sollen, möglich ist.

Nachfolgend wird die vorliegende Erfindung mit Bezug auf die beiliegenden Zeichnungen beispielhaft näher erläutert.

Es zeigen:
- FIG 1: ein Anlagenlayout zum Simulieren einer Fertigungsanlage mit mobilen elektronischen Geräten als zusammengestellte Produktionsmodule,
- FIG 2: zwei nebeneinander angeordnete Produktionsmodule zur Veranschaulichung der betätigbaren Berührobjekte,
- FIG 3: eine schematische Ausgestaltung eines Produktionsmoduls in einen mobilen elektronischen Gerät mit einer Anzeigeeinheit,
- FIG 4: Beispiel für Aufbau, Daten- und Kommunikationsstruktur eines aus Produktionsmodulen aufgebauten Produktionssystems,
- FIG 5: detaillierteres Beispiel für eine Datenstruktur eines Produktionsmoduls,
- FIG 6: Beispiel für eine Kombination eines Roboters mit einem Transportmodul,
- FIG 7: schematische Struktur des in FIG 6 dargestellten Beispiels eines Produktionssystems,
- FIG 8: Beispiel für den Verfahrensablauf bei der Kopplung zweier Produktionsmodule,
- FIG 9: Beispiele für flächige Anordnungsstrukturen von Produktions-Modulen zu einem Produktionssystem,
- FIG 10: detailliertere schematische Darstellung des in FIG 9 dargestellten Produktionssystems mit quadratischer Anordnung.

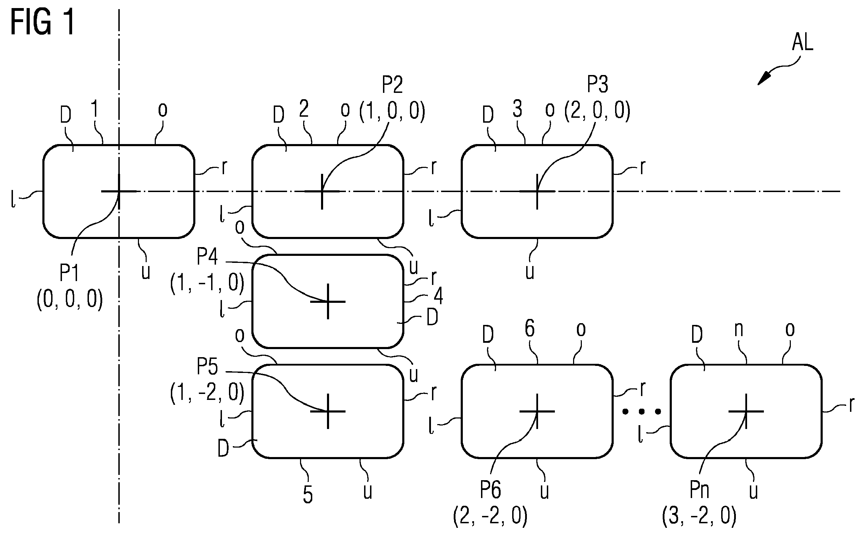

Gemäß FIG 1 ist ein Anlagenlayout AL zusammengestellt aus Produktionsmodulen 1, 2,..,n in einem Koordinatensystem mit einem Koordinatenursprung 0 dargestellt. Ein erstes Produktionsmodul 1 ist im Koordinatenursprung 0 mit einer ersten Position P1 (0,0,0) platziert. Das erste Produktionsmodul 1 sowie alle anderen Produktionsmodule 2,..,n weisen, ausgestaltet als mobile elektronische Geräte, neben einer Anzeigeeinheit d eine linke Anlegeseite l, eine rechte Anlegeseite r, eine obere Anlegeseite o und eine untere Anlegeseite u auf. An die rechte Anlegeseite r des ersten Produktionsmoduls 1 ist ein zweites Produktionsmodul 2 mit seiner linken Anlegeseite l angeordnet. Das zweite Produktionsmodul 2 nimmt somit in dem Koordinatensystem eine zweite Position P2 (1,0,0) ein. An die rechte Anlegeseite r des zweiten Positionsmoduls 2 ist ein drittes Positionsmodul 3 mit seiner linken Anlegeseite l angeordnet. Das dritte Positionsmodul 3 nimmt somit in dem Koordinatensystem eine dritte Position P3 (2,0,0) ein.

An die untere Anlegeseite u des zweiten Produktionsmoduls 2 ist ein viertes Produktionsmodul 4 mit seiner oberen Anlegeseite o angeordnet. Das vierte Positionsmodul 4 nimmt damit eine vierte Position P4 (1,-1,0) im Koordinatensystem ein. An die untere Anlegeseite u des vierten Positionsmoduls 4 ist ein fünftes Positionsmodul 5 mit seiner oberen Anlageseite o angeordnet. Das fünfte Positionsmodul 5 nimmt eine fünfte Position P5 (1,-2,0) ein. An die rechte Anlegeseite r des fünften Positionsmoduls 5 ist ein sechstes Positionsmodul 6 mit seiner linken Anlegeseite l angeordnet. Das sechste Produktionsmodul 6 nimmt eine sechste Position P6 (2,-2,0) im Koordinatensystem ein. Ausgehend vom sechsten Produktionsmodul 6 können jetzt noch in einer Reihe jeweils an die rechte Anlegeseite r der bereits platzierten Produktionsmodule beliebig viele Produktionsmodule Pn angelegt werden.

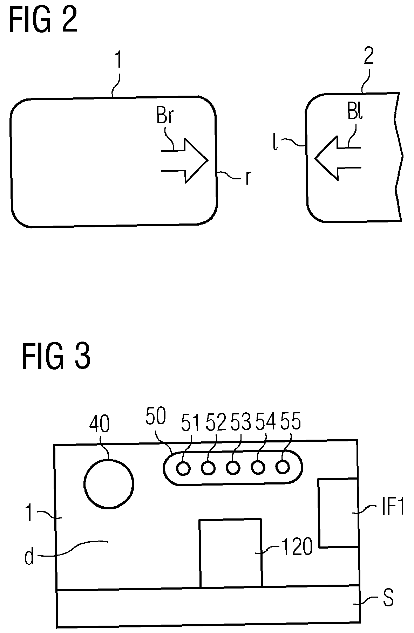

Mit dem so platzierten bzw. positionierten Produktionsmodulen 1,..,n kann durch aneinanderreihen z.B. von Tablet Computern ein Anlagenlayout AL, wie es später in der Realität existieren soll, zusammengestellt werden. Ausgehend von dem ersten Produktionsmodul 1, welches in den Koordinatenursprung 0 positioniert wurde, wird das zweite Produktionsmodul 2 als Nachbar zu dem ersten Produktionsmodul 1 mit seiner linken Anlegeseite l an die rechte Anlegeseite r des ersten Produktionsmoduls 1 angelegt. Die Anzeigeeinheiten D der Produktionsmodule 1,..,n zeigen abhängig von den fertigungsspezifischen Merkmalen der Produktionsmodule 1,..,n die möglichen Anlegeseiten l, r, o, u mittels Berührobjekten Br, Bl an (siehe FIG 2).

Gemäß der FIG 2 ist beispielhaft an dem ersten Produktionsmodul 1 und dem zweiten Produktionsmodul 2 das Anzeigen eines rechtsseitigen Berührobjektes Br und eines linksseitigen Berührobjektes Bl angedeutet, wobei das rechtsseitige Berührobjekt Br zum ersten Positionsmodul 1 gehört und das linksseitige Berührobjekt Bl zum zweiten Positionsmodul 2 gehört. Die Berührobjekte Br, Bl werden beispielsweise als aufeinander gerichtete Pfeile auf den Anzeigeeinheiten D der Produktionsmodule 1,..,n dargestellt. Da die Produktionsmodule 1,..,n beispielsweise als ein flacher Tablet PC ausgestaltet sind, weist die Anzeigeeinheit d des Tablet PC einen Touch Screen auf. Beim Betätigen beider Berührobjekte Br, Bl von den jetzt zueinander gewandten Anlegeseiten l, r wird die zweite Position P2 des zweiten Produktionsmoduls 2 bezogen auf den Koordinatenursprung 0 festgelegt. Für die Festlegung der weiteren Positionen P3,..,Pn werden an den möglichen Anlegeseiten l, r, o, u der restlichen Produktionsmodule P3,..,Pn jeweils in den Anzeigeeinheiten D auch wiederum Berührobjekte dargestellt. Ein Planer des zu simulierenden Anlagenlayout AL muss nun auf den Touch Screens der Produktionsmodule 1,..,n die jeweils zueinander gerichteten Anlegeseiten l, r, o, u, bei welchen die Berührobjekte Br, Bl auftauchen berühren und legt damit die Positionen jedes einzelnen Produktionsmoduls bezogen auf den Koordinatenursprung 0 fest.

Gemäß FIG 3 ist ein Produktionsmodul 1 mit schematischem Aufbau dargestellt. Das Produktionsmodul 1 weist eine Simulationssoftware S auf, wobei die Simulationssoftware S auf eine erste Selbstbeschreibungs-Information 120 zugreifen kann. In dem ersten Produktionsmodul 1 ist ein Produktmodell 40 vorhanden, welches nach Bearbeitung an weitere Produktionsmodule weitergereicht werden kann. Um durch Benutzereingaben das Produktionsmodul 1 auf fertigungsspezifische Merkmale des dieses Gerät repräsentierenden Produktionsmoduls anzupassen, ist ein Feld für Benutzereingaben 50 vorhanden. Das Feld für Benutzereingaben 50 weist einen ersten Touchbutton 51, beispielsweise zur Einstellung eines Roboters, einen zweiten Touchbutton 52, beispielsweise zur Einstellung eines Transportbandes, einen dritten Touchbutton 53, beispielsweise zum Einstellen eines Bohrers, einen vierten Touchbutton 54, beispielsweise zum Einstellen eines Montagesegments und einen fünften Touchbutton 55, beispielsweise zum Einstellen eines Gerätes auf einen Textbetrieb, auf.

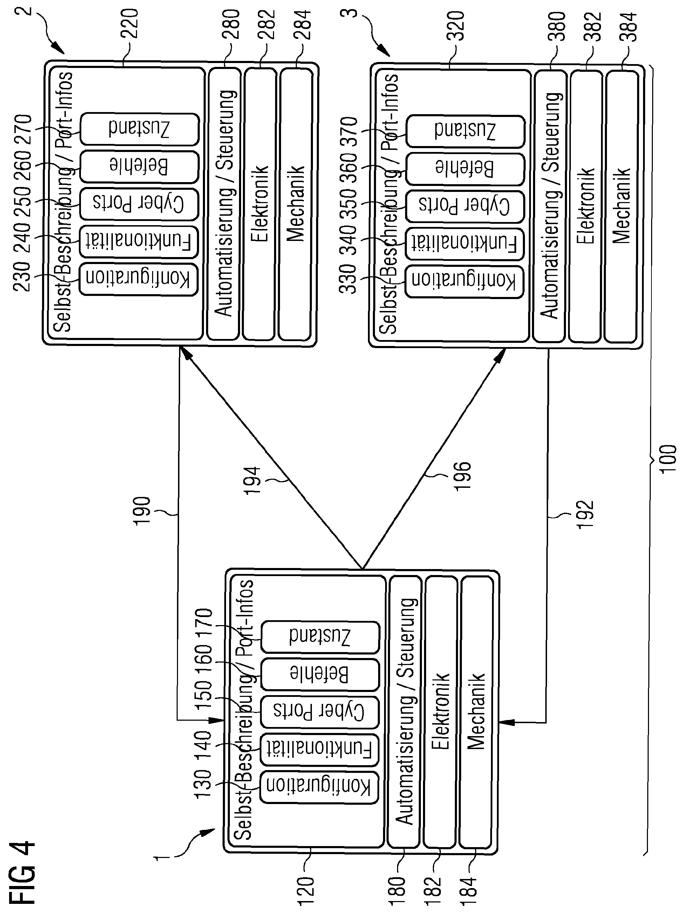

FIG 4 zeigt einen schematischen Aufbau einer Kopplung dreier Produktionsmodule 1, 2, 3.

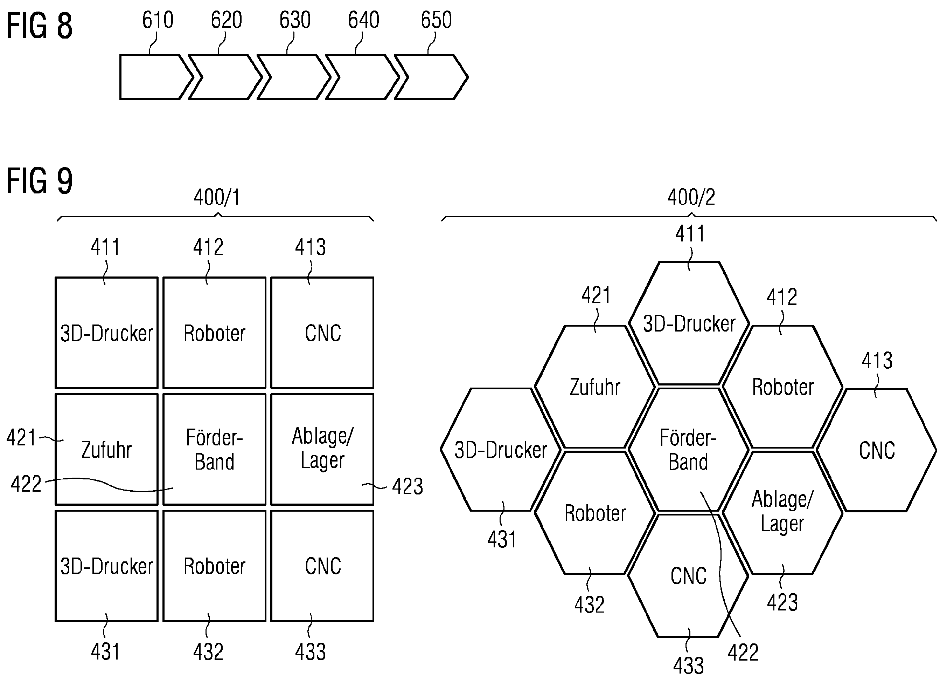

Für jedes der Produktionsmodule 1, 2, 3 ist schematisch ein Aufbau der Produktionsmodule dargestellt. Dabei umfassen die Produktionsmodule 1, 2, 3 jeweils einen Selbstbeschreibungs-Information 120, 220, 320, wobei in einem Speicherbereich der Selbstbeschreibungs-Informationen 120, 220, 320 der jeweiligen Produktionsmodule 1, 2, 3 jeweils eine Port-Informationen 150, 250, 350 bezüglich im jeweiligen Modul vorliegenden, so genannten "Cyber-Physical-Ports", gespeichert ist. Die in FIG 4 dargestellten Port-Informationen 150, 250, 350 sind Beispiele für Port-Informationen gemäß der vorliegenden Beschreibung. Weiterhin umfasst der Speicherbereich 120, 220, 320 der jeweiligen Produktionsmodule 1, 2, 3 jeweils eine Konfigurationsinformation 130, 230, 330 bezüglich einer funktionalen sowie elektronischen, mechanischen und kommunikativen Konfigurationen und sowie Eigenschaften des jeweiligen Moduls. Weiterhin umfasst der Speicherbereich der Selbstbeschreibungs-Informationen 120, 220, 320 der jeweiligen Produktionsmodule 1, 2, 3 eine Funktionalitäts-Beschreibung 140, 240, 340 des jeweiligen Moduls, eine Beschreibung verfügbarer Befehle 160, 260, 360, sowie eine oder mehrere Zustandsinformationen 170, 270, 370 bezüglich des jeweiligen Moduls 110, 210, 310. Die Konfigurationsinformationen 130, 230, 330, die Service-Information-Beschreibungen 140, 240, 340 bzw. die Funktionalität, die Beschreibungen verfügbarer Befehle 160, 260, 360 und Zustandsinformationen 170, 270, 370 sind jeweils Beispiele für Selbstbeschreibungs-Informationen gemäß der vorliegenden Beschreibung. Die vorstehend genannten Selbstbeschreibungs- beziehungsweise Port-Informationen werden im Zusammenhang mit FIG 8 noch beispielhaft weiter erläutert.

Weiterhin umfasst jedes der Produktionsmodule 1, 2, 3 eine Automatisierungs- und/oder Steuereinrichtung 180, 280, 380 zur Simulation der Automatisierung oder Steuerung beziehungsweise der Handhabung der verschiedenen Funktionalitäten und Services, welches das jeweilige Produktionsmodul 1, 2, 3 zur Verfügung stellt. Weiterhin ist in jedem der Produktionsmodule 1, 2, 3 eine oder mehrere Simulationsmodule für Elektronikbaugruppen oder -module 182, 282, 382 vorhanden, welche den Betrieb beispielsweise von Aktoren sowie anderen elektronischen, optischen und sonstigen Einrichtungen simulieren. Zudem umfasst jedes der Produktionsmodule 1, 2, 3 Simulationsmodelle für mechanische Elemente 184, 284, 384.

Die in FIG 4 dargestellten Pfeile 190, 192, 194, 196 stellen einen beispielhaften Kommunikationsablauf bei Änderungen im ersten Produktionsmodul 1 dar. Dazu registriert sich beispielsweise das zweite Produktionsmodul 2 über eine entsprechende Nachricht 190 beim ersten Produktionsmodul 1 als "Subscriber", d.h., als mit dem ersten Produktionsmodul 1 verbundenes Modul. Dieses Registrieren kann beispielsweise bei der Kopplung beziehungsweise unmittelbar nach der Kopplung beider Module oder auch später erfolgen. In gleicher Weise registriert sich das dritte Produktionsmodul 3 über eine entsprechende "Subscriber"-Nachricht 192 beim ersten Produktionsmodul 1. Bei Änderungen im ersten Produktionsmodul, beispielsweise einer Zustandsänderung von einem Normal-Zustand zu einem Stopp-Zustand, welcher dann in den ZustandsInformationen 170 des ersten Produktionsmoduls 1 gespeichert wird, schickt dann das erste Produktionsmodul 1 eine entsprechende Änderungsnachricht 194 an das zweite Produktionsmodul 210 und auch eine entsprechende Nachricht 196 an das dritte Produktionsmodul 3. Auf diese Weise sind das zweite und dritte Produktionsmodul 2, 3 über die Zustandsänderung im ersten Produktionsmodul 1 informiert und können dies beispielsweise bei der Koordination einer Produktionskette oder einer gemeinsamen Bearbeitung oder Behandlung eines Produkts berücksichtigen.

Über ein derartiges Kommunikationsschema kann im Prinzip dafür gesorgt werden, dass bei Änderungen in einem der Produktionsmodule 1, 2, 3 jeweils die damit unmittelbar oder auch mittelbar verbundenen Produktionsmodule informiert sind und dies im Rahmen des Zusammenwirkens innerhalb des Anlagenlayouts AL berücksichtigt werden kann. Ein entsprechender "Subscription"-Vorgang kann dann sinngemäß genauso vom ersten und dritten Produktionsmodul 1, 3 zum zweiten Produktionsmodul 2 beziehungsweise vom ersten und zweiten Produktionsmodul 1, 2 zum dritten Produktionsmodul 3 eingerichtet sein, so dass sich über einen solchen Mechanismus alle drei der in FIG 4 dargestellten Produktionsmodule 1, 2, 3 gegenseitig über Änderungen jeweils entsprechend informieren.

Die Produktionsmodule 1, 2, 3 können dabei beispielsweise als so genannte "Cyber-Physical-Modules" (CPM), oder auch als so genannte "Cyber-Physical-Production-Modules" (CPPM) nachgebildet werden.

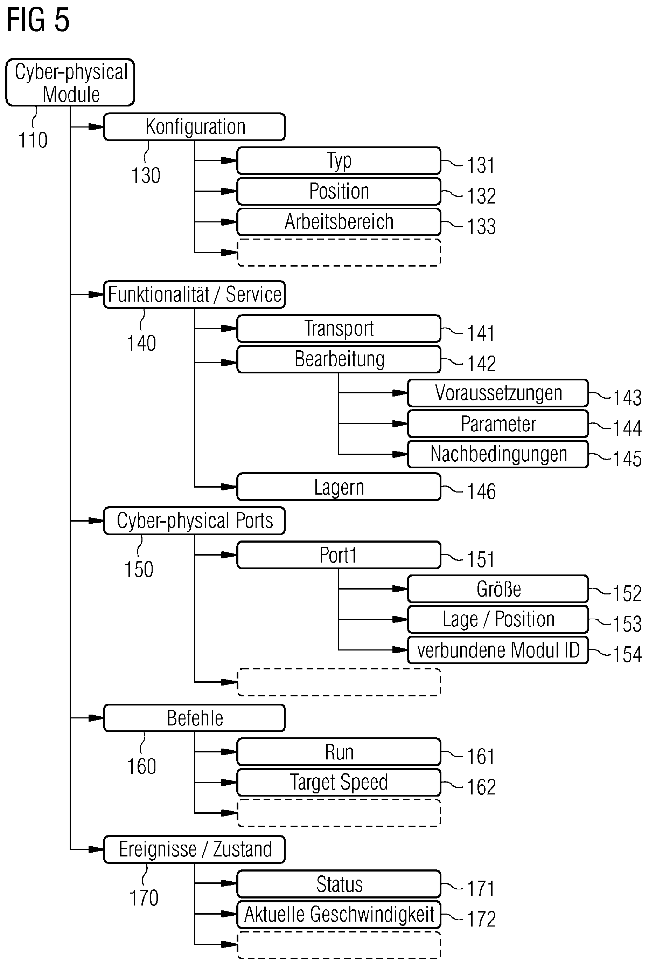

FIG 5 zeigt das Beispiel für einen schematischen Aufbau der in FIG 4 dargestellten Selbstbeschreibungs-Information 120 des ersten Produktionsmoduls 1. Der Speicher der Selbstbeschreibungs-Information 120 umfasst eine Konfigurationsinformation 130, in der beispielsweise ein Modultyp 131, eine geometrische Lage oder Anordnung 132 des Moduls oder von Funktionselementen des Moduls sowie ein Arbeitsbereich 133 des Moduls 110 gespeichert ist. Dabei kann der "Typ" 131 beispielsweise aus einer entsprechende Kennung oder auch einer oder mehreren Funktionsbezeichnungen bestehen beziehungsweise derartige Informationen umfassen. In der Information über den Arbeitsbereich 133 kann beispielsweise ein räumlicher Bereich beschrieben werden, in welchem Produkte im entsprechenden Produktionsmodul 1 vorhanden sein können beziehungsweise, innerhalb welchem diese Produkte bewegt werden können. So kann beispielsweise ein Arbeitsbereich eines Transportbandes ein entlang des Transportbandes sich längs erstreckender Volumenbereich sein, in welchem das Produkt mit Hilfe des Transportbandes bewegt werden kann. Bei einer Bearbeitungsmaschine kann der Arbeitsbereich beispielsweise der Volumenbereich sein, in welchem ein Bearbeitungselement der Maschine auf ein darin befindliches Produkt einwirken kann. Entsprechende Arbeitsbereiche lassen sich für weitere Funktionalitäten im Rahmen der vorstehend beschriebenen Produktionsfunktionen ermitteln.

Weiterhin umfasst der Speicherbereich der ersten Selbstbeschreibungs-Information 120 des ersten Produktionsmoduls 1 eine Funktionalitäts- und Service-Information 140, wobei diese Information weitere Informationen zu Transportmöglichkeiten 141 mit dem Modul, Bearbeitungs-Möglichkeiten 142 des Moduls oder auch Lagermöglichkeiten 146 zur Ablage oder Speicherung von Produkten oder Materialien umfasst. Die Bearbeitungsinformationen 142 können beispielsweise weiterhin Informationen zu Voraussetzungen zur Verwendung der entsprechenden Bearbeitungsfunktionen 143, entsprechende Parameter, welche die Bearbeitungsfunktionen näher kennzeichnen 144 und/oder auch Nachbedingungen 145 umfassen, welche für eine Weiterbehandlung des Produkts nach der Bearbeitung charakterisierend beziehungsweise notwendig sind.

Ganz allgemein kann ein Produktionsmodul gemäß der vorliegenden Beschreibung jeweils auch mehrere der genannten Produktions-Funktionalitäten umfassen, wobei dann beispielsweise in einer entsprechenden Speichereinrichtung für jede der Funktionalitäten eine Information abgelegt werden kann beziehungsweise abgelegt ist. So kann beispielsweise ein Produktionsmodul mehrere Transportfunktionalitäten, beispielsweise über ein oder mehrere Transportbänder oder Roboterarme beziehungsweise eine Kombination davon aufweisen, verschiedene Bearbeitungsfunktionen aufweisen, sowie auch verschiedene Lagerungsmöglichkeiten aufweisen.

In der Speichereinrichtung der Selbstbeschreibungs-Information 120 des Produktionsmoduls 1 ist weiterhin eine Port-Information 150 über einen so genannten "Cyber-Physical-Port" zu einem weiteren Produktionsmodul gespeichert. Dieser "Cyber-Physical-Port" kann sowohl Informationen über eine funktionale Zusammenarbeit von gekoppelten Produktionsmodulen, als auch Informationen über in den derart gekoppelten Modulen verfügbare Funktionalitäten umfassen.

So enthält die Port-Information 150 beispielsweise Informationen über einen ersten "Cyber-Physical-Port" 151 zu einem benachbarten, gekoppelten Produktionsmodul, beispielsweise dem zweiten Produktionsmodul 2 gemäß FIG 4. Dabei ist in der entsprechenden Portinformation 151 beispielsweise eine Information über eine Größe des Wechselwirkungs- oder Übergabebereichs 152 zum zweiten Produktionsmodul 2, eine Lage oder Position des Übergabebereichs 153, sowie einer Kennung 154 des verbundenen Moduls, enthalten. Weiterhin kann beispielsweise eine Information über eine Funktionalität des zweiten Produktionsmoduls 2 sowie Informationen über Funktionalitäten von weiteren, mit dem zweiten Produktionsmodul 2 verbundenen Produktionsmodulen in der entsprechenden Portinformation 151 gespeichert sein. Würde vom ersten Produktionsmodul 1 eine weitere unmittelbare Verbindung zu einem weiteren Produktionsmodul existieren, beispielsweise dem dritten Produktionsmodul 3 gemäß FIG 4, so würde auch für diesen entsprechenden "Cyber-Physical-Port" zum dritten Produktionsmodul 3 eine entsprechende Port-Information in der allgemeinen Port-Information 150 abgelegt.

Weiterhin enthält der Speicherbereich der Selbstbeschreibungs-Information 120 des ersten Produktionsmoduls 1 eine Information 160 über im Produktionsmodul verwendbare Befehle oder Kommandos zu dessen Steuerung. So ist in diesem Befehls-Speicher 160 beispielsweise eine Information zu einem Run-Befehl 161 oder auch zu einem Target-Speed-Befehl 162 niedergelegt, mit welchen diese Befehle mit entsprechenden verwendbaren Parametern definiert sind und von einem Benutzer des Systems zum Einrichten einer Steuerung für das erste Produktionsmodul 1 ausgelesen werden können.

Im Ereignis-/Zustandsinformations-Bereich 170 in der Speichereinrichtung 120 des ersten Produktionsmoduls 1 sind beispielsweise Informationen über den aktuellen Status des Produktionsmoduls 171 sowie beispielsweise über die aktuelle Geschwindigkeit eines Motors des Moduls 172 abgelegt.

Die Gesamtheit der im ersten Produktionsmodul 1 abgelegten Informationen gemäß FIG 5 ermöglicht es, auch mit relativ geringer Einwirkung eines Benutzers, oder auch ohne Einwirkung eines Benutzers, eine Simulation der Zusammenarbeit mehrerer derartiger Produktionsmodule zu organisieren und einen gemeinsamen Produktionsablauf solcher gekoppelter Produktionsmodule in einem Anlagenlayout AL sichtbar zu machen. Über die genannten Informationen ist es einem an ein solches Produktionsmodul gekoppeltes weiteres Modul möglich, sowohl Informationen über Funktionalitäten über die Geometrie, als auch über den Zustand und die Ansteuer- beziehungsweise Steuermöglichkeiten eines Moduls sowie die Kopplungsmöglichkeiten an das Modul zu erkennen und dies beispielsweise bei einem halbautomatisierten oder auch automatisierten Planungsprozess zu berücksichtigen.

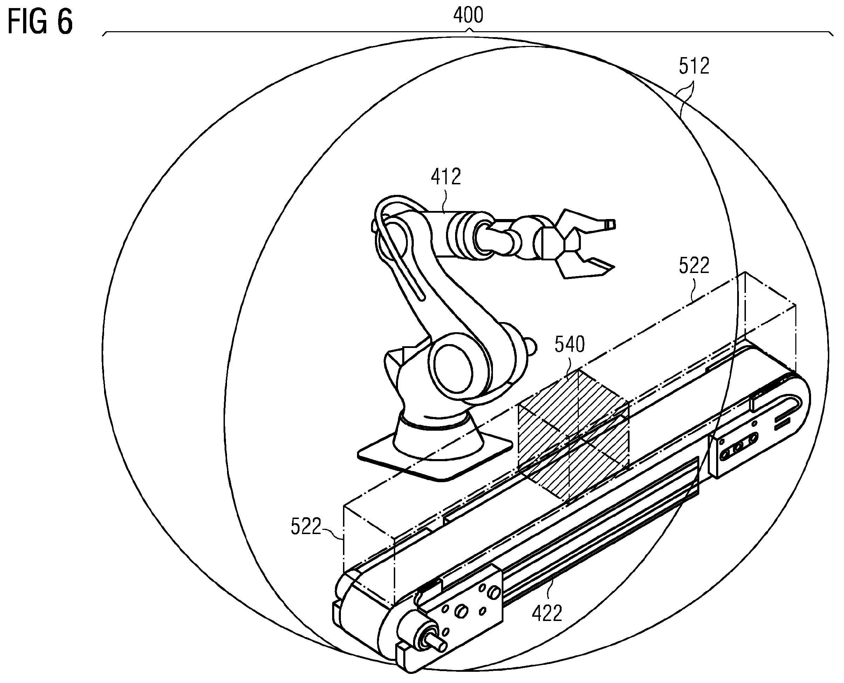

FIG 6 stellt einen kleinen Ausschnitt aus einem größeren Produktionssystem 400 dar, wobei dieser Ausschnitt einen Roboter 412 sowie ein Transport- oder Förderband 422 umfasst. Dabei ist in FIG 6 zum Transport- oder Förderband 422 ein Arbeitsbereich 522 dargestellt, innerhalb dessen Volumenbereich ein Produkt transportierbar ist. Die Information über den Arbeitsbereich 522 kann beispielsweise innerhalb einer entsprechenden Konfigurations-Information in einem Speicher des Transportbands 422 abgelegt sein. Weiterhin ist ein Arbeitsbereich 512 des Roboterarms 412 durch kreisförmige Linien aufgespannt, welche einen kugelförmigen Arbeitsbereich für den Roboterarm 412 kennzeichnen. In FIG 6 ist auch ein Wechselwirkungsbereich 540 zwischen den Transportband 422 und dem Roboterarm 412 dargestellt, innerhalb dessen sich ein Produkt befinden muss, damit es vom Roboterarm 412 an das Transportband 422 übergeben werden kann oder vom Roboterarm 412 vom Transportband 422 abgenommen werden kann.

Die Information über diesen Wechselwirkungsbereich kann dann beispielsweise in einer Speichereinrichtung des für das Transportband 422 konfigurierte Produktionsmodul und/oder in einem Speicherbereich des für den Roboterarm 412 konfigurierte Produktionsmodul abgelegt sein. Die Ermittlung des Wechselwirkungsbereichs 540 kann beispielsweise bei der Kopplung des Roboterarms 412 mit dem Transportband 422 erfolgen. Wird die geometrische Lage zwischen Roboterarm 412 und dem Transportband 422 geändert, so kann auch ein geänderter Wechselwirkungsbereich 540 ermittelt werden. Die Ermittlung des Wechselwirkungsbereichs kann beispielsweise wie in der vorliegenden Beschreibung näher erläutert vorgenommen werden.

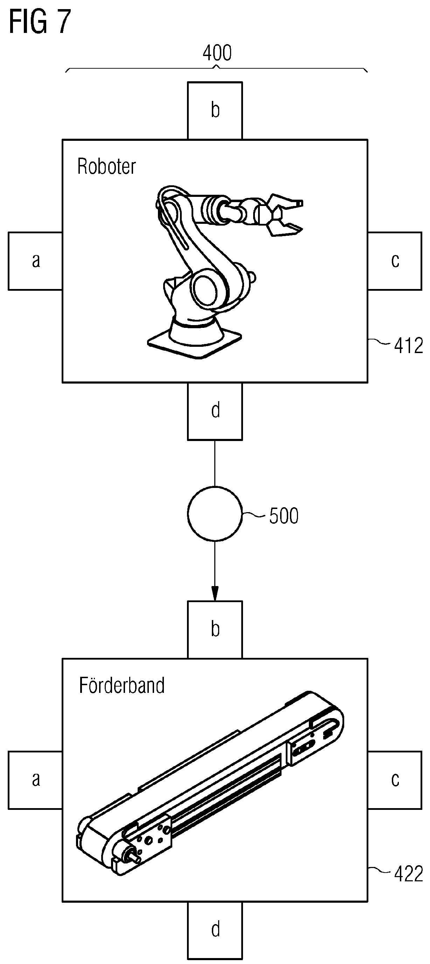

Die Kopplung des Transportbands 422 an den Roboterarm über den Wechselwirkungsbereich 450 ist beispielsweise als ein "Cyber-Physical-Port" des Transportbands 422 zum Roboterarm 412 beschreibbar. Genauso ist aus Sicht des Roboterarms 412 die Kopplung zum Transportband 422 über den Wechselwirkungsbereich 540 als entsprechender "Cyber-Physical-Port" beschreibbar. Dies ist in FIG 7 in schematisierter Weise dargestellt.

FIG 7 zeigt eine schematische Darstellung des Roboterarms 412 sowie eine schematische Darstellung des Transport- oder Förderbandes 422. Dabei ist der "Cyber-Physical-Port" 412/d aus der Sicht des Roboters 412 zum Transportband 422 als Quadrat mit Bezeichnung "d" in FIG 7 gekennzeichnet. Genauso ist der "Cyber-Physical-Port" 422/b aus Sicht des Transportbands 422 zum Roboter 412 als Quadrat mit "b" an der schematischen Darstellung des Förderbandes 422 dargestellt. Weiterhin sind in FIG 7 weitere potentielle "Cyber-Physical-Ports" des Roboterarms 412, 412/a, 412/b, 412/c dargestellt, welche entsprechende "Cyber-Physical-Ports" 412/a, 412/b, 412/c zu in FIG 7 nicht dargestellten benachbarten Modulen darstellen, beziehungsweise entsprechende Speichereinrichtungen für entsprechende Portinformationen symbolisieren. In gleicher Weise sind auch für das Förderband 422 weitere "Cyber-Physical-Ports" 422/a, 422/c, 422/d dargestellt, welche potentielle "Cyber-Physical-Ports" zu weiteren, nicht in FIG 7 dargestellten Produktionsmodulen, oder auch Speicherbereiche für entsprechende Portinformationen symbolisieren.

Weiterhin ist in ein vom Roboter 412 an das Transportband 422 zu übergebendes Produkt 500 dargestellt. In der schematischen Darstellung von ist dies derart dargestellt, dass das Produkt 500 quasi in den "Cyber-Physical-Port" 412/d des Roboters 412 zum Förderband 422 durch den Roboter verbracht wird und dann im "Cyber-Physical-Port" 422/b des Förderbands übernommen wird um dann z.B. vom Förderband 422 weiter transportiert zu werden.

Dabei beschreiben beide Portinformationen 412/d und 422/d zumindest unter anderem denselben räumlichen Wechselwirkungsbereich, um eine entsprechende Produkt-Übergabe realisieren zu können.

FIG 8 zeigt ein Beispiel für einen schematischen Ablauf bei der Kopplung zweier Produktionsmodule, die beispielsweise gemäß der vorliegenden Beschreibung oder insbesondere entsprechend den in FIG 4 bis 7 dargestellten Produktionsmodule ausgebildet und eingerichtet sein können.

In einem ersten Schritt 610 wird ein neues Produktionsmodul zu einem bereits platzierten Produktionsmodul assoziiert. Diese Assoziierung 610 kann beispielsweise über einen manuellen Benutzereingriff, z.B. über einen entsprechenden Touchscreen der Module oder eine entsprechende Netzwerkverbindung, ausgelöst werden, oder auch durch entsprechende Abstands- oder ähnlichen Sensoren, welche das neue Produktionsmodul als benachbart zum bereits platzierten Produktionsmodul erkennen. Die assoziierten Produktionsmodule werden somit Nachbarn und melden sich über entsprechende Kommunikationsmittel als solche beim jeweils anderen Produktionsmodul an. Durch diese gegenseitige Anmeldung kann zumindest unter Anderem bewirkt werden, dass entsprechende Änderungen bei jedem der Produktionsmodule dem jeweils anderen Produktionsmodul mitgeteilt werden. Dies kann beispielsweise über einen Subscription-Kommunikations-Modus, wie er im Zusammenhang mit FIG 4 erläutert wurde, erreicht werden.

In einem Ausrichtungsschritt 620 wird dann eine topologische Ausrichtung der Positionen und Arbeitsbereiche der Produktionsmodule erreicht oder eingerichtet. Dies kann beispielsweise unter Verwendung eines Koordinatensystems des bereits installierten Moduls geschehen. Abhängig von der verfügbaren Technologie kann dies vollautomatisiert geschehen (z.B. mit Hilfe eines entsprechenden Positionierungssystems und so genannter "Near Field Communication" oder auch RFID-Technologie). Weiterhin können die Module beispielsweise auch einen Bediener, z.B. über ein entsprechendes Bedien-Panel, dazu anleiten, wie z.B. das neue Modul bewegt oder ausgerichtet werden muss.

Eine derartige Ausrichtung lässt sich einfacher erreichen, wenn für das gesamte Produktionssystem eine entsprechende Gitter- oder Wabenstruktur festgelegt wird, wie dies im Zusammenhang mit FIG 9 näher erläutert wird.

In einem Berechnungsschritt 630 erfolgt eine automatische Berechnung der "Cyber-Physical-Ports", welcher unter anderem die Berechnung eines Schnittvolumens zwischen den Arbeitsbereichen beider Module umfasst. Ein solches Schnitt-Volumen ist ein Beispiel für einen Wechselwirkungs-Bereich gemäß der vorliegenden Beschreibung. Die Informationen über die jeweiligen Arbeitsbereiche der einzelnen Module sind in den jeweiligen Modulen abgelegt und werden durch diese auch an andere Module übermittelt. Auf diese Weise kann das bereits installierte Modul vom neuen Modul dessen Arbeitsbereich erfahren und über die Kenntnis des eigenen Arbeitsbereichs und eine relative Positionierung dann das Schnittvolumen berechnen. Ergibt die Berechnung, dass sich beide Arbeitsbereiche nicht überlappen, so können die entsprechenden Produktionsmodule im Regelfall nicht einfach funktional miteinander gekoppelt werden.

In einem vierten Informations-Austausch-Schritt 640 erfolgt ein Austausch der entsprechenden Serviceinformationen bezüglich der Eigenschaften der jeweiligen Produktionsmodule an das andere Produktionsmodul. Über den in Zusammenhang mit FIG 4 bereits erläuterten Anmelde- und Veröffentlichungsmechanismus kann beispielsweise das bereits platzierte Produktionsmodul die Informationen bezüglich der Eigenschaften des neuen Produktionsmoduls übertragen bekommen, diese bei sich abspeichern und gegebenenfalls an weitere, mit dem bereits platzierte Produktionsmodul bereits verbundene Produktionsmodule weitergeben. Die Information über die Funktionalität beziehungsweise die Eigenschaften oder Kennung des neuen Produktionsmoduls kann dann beispielsweise in der Port-Information des platzierten Produktionsmoduls bezüglich des neuen Produktionsmoduls abgelegt werden.

In einem Abstimmungsschritt 650 erfolgt dann eine Abstimmung der Funktionalität des bereits installierten Produktionsmoduls mit dem neu platzierten Produktionsmodul, um ein Zusammenwirken der Funktionalitäten beider Produktionsmodule zu ermöglichen. Eine solche Abstimmung kann beispielsweise die Anpassung von Transportgeschwindigkeiten zweier miteinander verbundener Transportbänder oder auch die Anpassung einer Transportgeschwindigkeit an einen durchzuführenden Bearbeitungsprozess oder Ähnliches beinhalten. Nach Abschluss des Zusammenarbeitsschritts 650 können das neuen Produktionsmodul und das bereits platzierte Produktionsmodul in Bezug auf eine simulierte Bearbeitung und Produktion eines Produkts zusammenwirken.

FIG 9 stellt zwei Beispiele für räumliche regelmäßige Anordnungen des Produktionssystems 400 dar, wobei in FIG 9 nun das vollständige Produktionssystem 400 dargestellt ist. In einer ersten quadratischen Rasterstruktur des Produktionssystems 400/1 befindet sich jedes der Produktionsmodule innerhalb eines quadratischen Flächenbereichs. Das Produktionssystem 400 umfasst dabei zwei 3D-Drucker 411, 431, zwei Roboter 412, 432 sowie zwei CNC-Maschinen 413, 433 (CNC: Computerized Numerical Control). Weiterhin umfasst das Produktionssystem eine Zufuhr und Transporteinheit 421 für das Produkt, ein Transport- oder Förderband für das Produkt 422 sowie eine Transport- und Lagereinheit 423.

Durch die räumliche Ausgestaltung der jeweiligen Modulumrisse können die einzelnen Produktionsmodule so ausgebildet werden, dass sie sich vollständig innerhalb des entsprechenden quadratischen Flächenbereichs befinden und auch die Abstände zu den Rändern der jeweiligen quadratischen Zellen bekannt sind. Somit können bereits bei der Software-Entwicklung der einzelnen Produktionsmodule entsprechende mechanische Elemente zum Transport oder zur Bearbeitung der Produkte so vorbereitet sein, dass eine Zusammenwirkung mit einer benachbarten quadratischen Zelle möglich ist.

FIG 9 zeigt weiterhin eine alternative Ausgestaltung des Produktionssystems als hexagonales Waben-System 400/2, bei welchem die Produktionsmodule des Produktionssystems 400 in jeweiliger hexagonaler Zellenanordnung ausgebildet und zu einem Produktionssystem zusammengefügt sind.

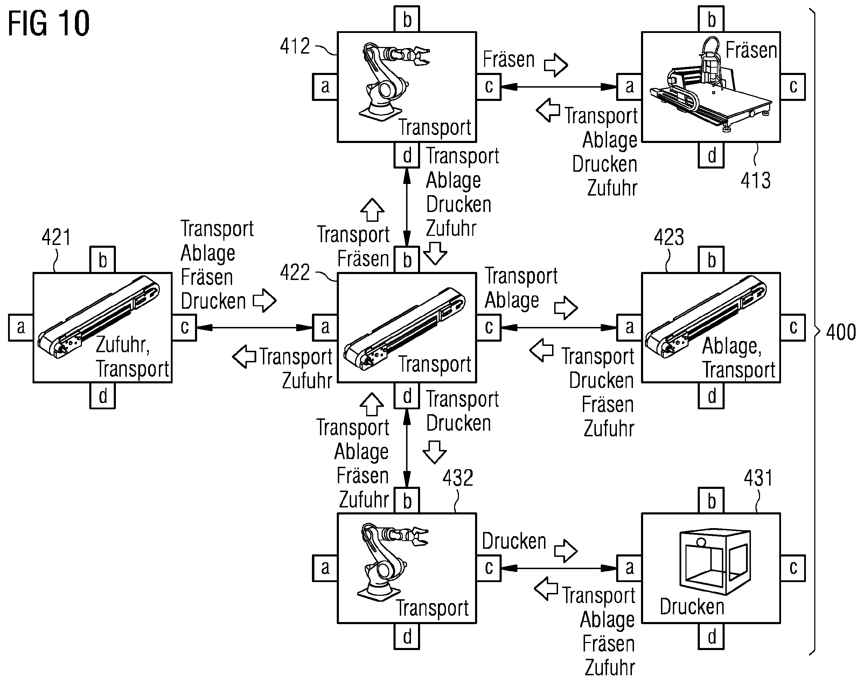

FIG 10 stellt das in FIG 9 dargestellte Produktionssystem 400 in einem Ausschnitt und einer funktionaleren Darstellung dar. In FIG 10 ist das Produktionssystem 400 im Gegensatz zu FIG 9 mit nur einem 3D-Drucker 431 sowie nur einer CNC-Fräse 413 dargestellt.

Die Darstellung der einzelnen Produktionsmodule in FIG 10 erfolgt symbolisch, wobei zu jedem der Produktionsmodule vier "Cyber-Physical-Ports" a, b, c, d als angehängte, kleine Quadrate dargestellt sind, welche einen möglichen oder auch existierenden "Cyber-Physical-Port" symbolisieren.

So hat beispielsweise das Transport- oder Förderband 422 vier existierende "Cyber-Physical-Ports" 422/a, 422/b, 422/c, 422/d. Dabei symbolisiert der "Cyber-Physical-Port" 422/a zum Zufuhr-Transport-Modul 421 den "Cyber-Physical-Port" zu diesem Modul. Die in Bezug auf diesen "Cyber-Physical-Port" 422/a gespeicherten Informationen umfassen auch sämtliche Funktionalitäten, welche über diesen "Cyber-Physical-Port" erreichbar sind. Diese erreichbaren Funktionalitäten sind in FIG 10 als Text neben das jeweiligen quadratische Symbol des "Cyber-Physical-Ports" geschrieben. So enthält der "Cyber-Physical-Port" 422/a unter Anderem die Information, dass über diesen Port, das heißt diese Verbindung, zum Zufuhr/Transport-Modul, die Funktionalitäten: "Transport" und "Zufuhr" erreichbar sind, welche vom entsprechenden Modul 421 ausgeführt werden können. Über den Port 422/b des Transportmoduls 422 zum Roboter 412 sind beispielsweise die Funktionalitäten "Transport" und "Fräsen" verfügbar, welche dann in der entsprechenden Portinformation 422/b abgelegt sind. Dabei wird die Funktionalität "Transport" vom Roboter 412 durchgeführt, während die Funktionalität "Fräsen" von der mit dem Roboter 412 verbundenen CNC-Fräse 413 durchgeführt wird.

Über den Port 421/c des Zufuhr-, Transport-Moduls 421 sind auf diese Weise beispielsweise alle Funktionalitäten (außer der eigenen) des Produktionssystems 400 verfügbar - also über die verschiedensten Wege die Funktionalitäten "Transport", "Ablage", "Fräsen" sowie "Drucken".

Auf diese Weise kann bei einem, in einem bestimmten Produktionsmodul befindlichen Produkt, und bekannten nächsten erforderlichen Arbeitsschritten jeweils durch Analyse der Portinformationen des jeweiligen Moduls bei Bedarf ein Produktionsablauf zur weiteren Produktion des Produkts bestimmt werden.

Die Organisation der einzelnen in den Ports abgelegten Funktionsinformationen über die verbundenen Produktionsmodule, kann beispielsweise über einen Informations-Verteilungsschritt, wie er beispielsweise in FIG 8 in Zusammenhang mit dem Schritt 640 erläutert wurde, erreicht werden. Über einen, wie beispielsweise in Zusammenhang mit FIG 4 erläuterten Änderungs-Weitergabe-Mechanismus können die jeweiligen Informationen dann aktuell gehalten werden.

## Patentansprüche

1. Verfahren zur Simulation einer industriellen Fertigungsanlage, wobei mehrere Produktionsmodule (1,..,n) zu einem Anlagenlayout (AL) der zu simulierenden Fertigungsanlage zusammen gestellt werden,
**dadurch gekennzeichnet, dass** als Produktionsmodule (1,..,n) tragbare, mobile elektronische Geräte mit einer Anzeigeeinheit (D) verwendet werden, wobei auf den Geräten eine Simulationssoftware (S) zum Ablauf gebracht wird, und das die Simulationssoftware (S) auf dem jeweiligen Gerät durch eine Benutzereingabe auf fertigungsspezifische Merkmale des dieses Gerät repräsentierenden Produktionsmoduls (1,..,n) angepasst wird, wobei ein erstes Produktionsmodul (1) durch eine Simulation auf einem ersten Gerät und ein zweites Produktionsmodul (2) durch eine Simulation auf einem zweiten Gerät repräsentiert wird, wobei zur Simulation eines Fertigungsablaufs dem ersten Produktionsmodul (1) ein computergeneriertes Produktmodell (40) übergeben wird und entsprechend der fertigungsspezifischen Merkmale des ersten Produktionsmoduls (1) eine erste Produktions-Funktion auf das Produktmodell (40) angewendet wird und das Produktmodell (40) an das zweite Produktionsmodul (2) übergeben wird, wobei in dem zweiten Produktionsmodul (2) auf das Produktmodell (40) eine zweite Produktions-Funktion angewendet wird.

2. Verfahren nach Anspruch 1, wobei jedes tragbare, mobile elektronische Gerät eine linke Anlegeseite (l), eine rechte Anlegeseite (r), eine obere Anlegeseite (o) und eine untere Anlegeseite (u) aufweist und das Anlagenlayout (AL) durch folgende Schritte festgelegt wird:
a) Positionieren des ersten Produktionsmoduls (1) an eine erste Position (P1), wobei durch diese erste Position (P1) des ersten Produktionsmoduls (1) ein Koordinatenursprung (0) gebildet wird,
b) abhängig von den fertigungsspezifischen Merkmalen des ersten Produktionsmoduls (1) werden auf der Anzeigeeinheit (D) des ersten Produktionsmoduls (1) mögliche Anlegeseiten (l, r, o, u) mittels eines Berührobjektes (Bᵣ, Bₗ) angezeigt,
c) Positionieren des zweiten Produktionsmoduls (2) als Nachbar zu dem ersten Produktionsmodul (1), wobei auf der Anzeigeeinheit (d) des zweiten Produktionsmoduls (2) abhängig von den fertigungsspezifischen Merkmalen des zweiten Produktionsmoduls (2) ebenfalls mögliche Anlegeseiten (l, r, o, u) mittels weiterer Berührobjektes (Bᵣ, Bₗ) angezeigt werden, anlegen einer durch ein Berührobjekt (Bₗ) markierten Anlegeseite (l, r, o, u) des zweiten Geräts an eine durch ein Berührobjekt (Bᵣ) markierten Anlegeseite (l, r, o, u) des ersten Geräts,
d) Betätigen beider Berührobjekte (Bᵣ, Bₗ) von den jetzt zueinander gewandten Anlegeseiten (l, r, o, u), wodurch eine zweite Position (P2) des zweiten Produktionsmoduls (2) bezogen auf den Koordinatenursprung (0) festgelegt wird,
f) Positionieren der weiteren Produktionsmodule (3,..,n) analog zu dem wie unter Schritt c) und d) beschrieben bis das gewünschte Anlagenlayout (AL) erreicht ist.

3. Verfahren nach Anspruch 1 oder 2, wobei zur Kopplung des ersten Produktionsmoduls (1) mit dem zweiten Produktionsmodul (2), im ersten Produktionsmodul (1) eine erste Selbstbeschreibungs-Information (120) bezüglich der fertigungsspezifischen Merkmale des ersten Produktionsmoduls (1) und im zweiten Produktionsmodul (2) eine zweite Selbstbeschreibungs-Information (220) bezüglich der fertigungsspezifischen Merkmale des zweiten Produktionsmoduls (2) gespeichert werden, und folgende Schritte durchgeführt werden:
- Koppeln des ersten und zweiten Produktionsmoduls (1, 2),
- Übertragen der zweiten Selbstbeschreibungs-Information (42) des zweiten Produktionsmoduls (2) an das erste Produktionsmodul (1), und
- Ermitteln einer ersten Port-Information (150, 250, 350) bezüglich der Kopplung mit dem zweiten Produktionsmodul (2) durch das erste Produktionsmodul (1) und Speicherung der ersten Port-Information im ersten Produktionsmodul (1).

4. Verfahren nach Anspruch 3, wobei nach der Kopplung des ersten und zweiten Produktionsmoduls (1, 2) weiterhin
- die erste Selbstbeschreibungs-Information (120) des ersten Produktionsmoduls (1) und/oder die erste Port-Information (150, 250, 350) an das zweite Produktionsmodul (2) übertragen wird, und
- durch das zweite Produktionsmodul (2) eine zweite Port-Information (150, 250, 350) bezüglich der Kopplung zum ersten Produktionsmodul ermittelt und im zweiten Produktionsmodul gespeichert wird.

5. Verfahren gemäß Anspruch 3 oder 4,
**dadurch gekennzeichnet, dass** die Ermittlung der ersten und/oder zweiten Port-Information die Ermittlung einer Information über einen räumlichen Wechselwirkungsbereich (540) des ersten Produktionsmoduls (1) mit dem zweiten Produktionsmodul (2) umfasst, wobei der Wechselwirkungsbereich (540) **dadurch gekennzeichnet ist, dass** sowohl die erste Produktions-Funktion des ersten Produktionsmoduls (1) als auch die zweite Produktions-Funktion des zweiten Produktions-Moduls (2) an dem Produktmodell (40) wirken kann, wenn es sich im Wechselwirkungsbereich (540) befindet.

6. Verfahren gemäß einem der Ansprüche 2 bis 5,
**dadurch gekennzeichnet, dass** die Selbstbeschreibungs-Information (120, 220, 320) umfasst:
- eine Service Information (140) bezüglich der Produktions-Funktion,
- eine Konfigurations-Information (130) bezüglich einer Lage und/oder Ausgestaltung des Produktionsmoduls,
- eine Fähigkeits-Information bezüglich verfügbarer Funktionen und Services des Produktionsmoduls, welche eine Information über die Produktions-Funktion umfasst,
- eine Befehls-Information (160) bezüglich vom Produktionsmodul ausführbarer Befehle und einstellbarer Parameter, und/oder
- eine Zustands-Information (170) bezüglich eines Arbeitszustands des Produktionsmoduls.

7. Verfahren nach einem der Ansprüche 1 bis 6, **dadurch gekennzeichnet, dass** wenn die Simulation einen Zielzustand erreicht hat, die während der Simulationen gewonnenen Parameter und die Einstellungen zumindest einer der folgenden Informationen:
- die Selbstbeschreibungs-Information (120, 220, 320) die Service Information (140),
- die Konfigurations-Information (130),
- die Fähigkeits-Information,
- die Befehls-Information (160),
- die Zustands-Information (170),
gespeichert werden und für eine spätere reale Produktion mit einer realen Fertigungsanlage und realen Produktionsmodulen genutzt werden.

8. Computerprogrammprodukt zur Simulation einer industriellen Fertigungsanlage, das auf einem computerlesbaren Medium gespeichert ist und Instruktionen aufweist, die die Schritte gemäß einem der Ansprüche 1 bis 7 für ein mobiles Gerät ausführen.
